Europäisches Patentamt

European Patent Office

Office européen des brevets

(11) Veröffentlichungsnummer: **0 493 741 A2**

## EUROPÄISCHE PATENTANMELDUNG

(21) Anmeldenummer: **91121639.8**

(22) Anmeldetag: **17.12.91**

(51) Int. Cl.5: **A61M 5/168**, G08B 29/18

(30) Priorität: **21.12.90 DE 9017231 U**

(43) Veröffentlichungstag der Anmeldung:
**08.07.92 Patentblatt 92/28**

(84) Benannte Vertragsstaaten:
**AT BE CH DE DK ES FR GB GR IT LI LU NL SE**

(71) Anmelder: **CRITIKON GmbH**
**Aspelohe 27a**
**W-2000 Norderstedt(DE)**

(72) Erfinder: **Witsch, Wolfgang**
**Aspelohe 27a**
**W-2000 Norderstedt(DE)**
Erfinder: **Ziel, Klaus-Dieter**
**Aspelohe 27a**
**W-2000 Norderstedt(DE)**

(74) Vertreter: **Richter, Werdermann & Gerbaulet**
**Neuer Wall 10**
**W-2000 Hamburg 36(DE)**

(54) **Schwerkraftinfusionsregelgerät.**

(57) Das Schwerkraftinfusionsregelgerät zur Erkennung und Herausfilterung zeitlich begrenzter Behinderungen des Infusionsflusses ohne eine Auslösung einer Alarmmeldung besteht aus einem Gerätegehäuse (6) mit einer Aufnahmekammer für eine Tropfenkammer (1), einer im Bereich der Tropfenkammer (1) angeordneten fotoelektrischen Tropfenfassungseinrichtung (7) mit einem Verhältniszähler (23) zur Registrierung der Tropfrate pro Zeiteinheit und einer elektronischen überwachungseinrichtung mit Bedienungselementen sowie einer Regelmechanik, wobei der Verhältniszähler (23) mit einer Torschaltung und diese mit einer internen Geräteüberwachungseinheit verbunden sind, an die durch die Torschaltung bei Unter- oder überschreiten einer eingestellten Tropfrate ein Signal abgebbar ist, welches vorgebbare Funktionsüberwachungszyklen auslost und wobei die Geräteüberwachungseinheit mit einem Vorwahlzähler verbunden ist, der erst bei überschreiten einer vorgebbaren Anzahl von Funktionsüberwachungszyklen ein Signal an eine Warneinrichtung abgibt.

Fig.1

Die Erfindung betrifft ein Schwerkraftinfusionsregelgerät, das aus einem Gerätegehäuse mit einer Aufnahmekammer für eine Tropfenkammer besteht, eine im Bereich der Tropfenkammer angeordnete fotoelektrische Tropfenerfassungseinrichtung mit einem Verhältniszähler zur Registrierung der Tropfrate pro Zeiteinheit und eine elektronische Überwachungseinrichtung mit Bedienungselementen sowie eine Regelmechanik aufweist.

Solche Schwerkraftinfusionsregelgeräte werden beispielsweise in der EP 0 154 163 beschrieben. Dieses Schwerkraftinfusionsregelgerät besitzt einen monolithischen Aufbau, wobei in einem einzigen Gerätgegehäuse eine fotoelektrische Tropfenerfassungseinrichtung, elektronische Einrichtungen mit Bedienungselementen und eine Regelmechanik eingeschlossen sind. Das Gerätegehäuse kann direkt an eine Tropfenkammer angeklemmt werden, deren Einstichdorn mit hinreichender Haftfähigkeit gegen Zugbelastung in dem Gummistopfen des Infusionsbehälters eingeklemmt ist. Das Gerätgehäuse selbst weist in seinem Vorderteil eine den Konturen der Tropfenkammer entsprechend ausgebildete Ausnehmung zur Aufnahme der Tropfenkammer auf, die beispielsweise frontal einsetzbar ist. An dem Gerätegehäuse ist ferner ein U-förmiger, unter der Einwirkung einer Feder stehender Hebel angeordnet, dessen beide freie Schenkel obenseitig und untenseitig an dem Gerätegehäuse verschwenkbar gelagert sind und dessen die beiden genannten Schenkel miteinander verbindender Steg an der Vorderseite des Gehäuses als Handhabe liegend ist, wobei der obere Schenkel des Hebels in einer Ausnehmung mit einer der Tropfenkammer entsprechenden Formgebung zur Klemmung und Halterung der Tropfenkammer versehen ist, während der untere Schenkel des Hebels ein mittels des Hebels verschwenkbares, zur Anlage an dem eingelegten Infusionsschlauch Dringbares Widerlager zum ungehinderten Einlegen des mit der Tropfenkammer verbundenen Infusionsschlauches bei gedrückter Hebelstellung und geöffnetem Widerlage und für die Quetschung des Infusionsschlauches durch ein Regelschwert bei Normal-Hebelstellung aufweist. Hierbei steht der Hebel gegen ein unbeabsichtigtes Betätigen mit einer knopfartigen, an dem oberen Hebelschenkel anliegenden Sperre in Wirkverbindung, die vor dem Verschwenken des Hebels betätigbar ist. Die genannte Sperre dient einerseits als Schutz vor einer unbeabsichtigten Betätigung mit der wahrscheinlichen Folge eines Freiflusses der Infusionslösung und gleichzeitig durch Betätigung eines Mikroschalters als Meldeelement für die richtige Lage des Hebels und demzufolge auch für die korrekte Montage der Tropfenkammer und des Infusionsschlauches, damit die elektronische Einrichtung im Fall von Bedienungsfehlern Alarm melden kann.

Bei den nach dem Stand der Technik bekannten Geräten steht in der Tropfenkammer eine konstante Flüssigkeitssäule an. Eine Unterbrechung des Infusionsflusses, z.B. durch eine leere Infusionsflasche, einen geknickten Schlauch, einen geschlossenen Dreiwegehahn, eine geknickte Kanüle, eine verlegte Kanüle oder einen nicht ausreichenden Infusionsdruck oder bei Zuschaltung von parallel laufenden Infusionen kommt bei Schwerkraftsystemen in der Praxis aufgrund des geringen Infusionsdruckes von ca. 80 cm Wassersäule häufig vor. Die nach dem Stand der Technik bekannten Schwerkraftinfusionsregelgeräte geben in diesen Fällen schon nach kurzer Zeit Alarm. Handelt es sich bei der Unterbrechung des Infusionsflusses nur um eine kurzzeitig auftretende Infusionsunterbrechung, die regeltechnisch binnen kurzer Zeit aufgehoben werden kann und bei der kein Grund für das Eingreifen bzw. sofortige Handeln des Pflegepersonals besteht, bemüht sich dieses vergeblich, um beim Patienten den geregelten Infusionsfluß zu überprüfen. Diese Überprüfung ist nachteiligerweise mit einer Beunruhigung des Patienten sowie weiterer, im Raum befindlicher Patienten verbunden.

Es ist daher Aufgabe der vorliegenden Erfindung, das eingangs genannte Schwerkraftinfusionsregelgerät dahingehend zu verbessen, daß zeitlich begrenzte Behinderungen (Artefakte) des Infusionsflusses als solche erkannt und herausgefiltert werden, ohne daß eine Alarmmeldung ausgelöst wird.

Diese Aufgabe wird durch das im Anspruch 1 beschriebene Schwerkraftinfusionsregelgerät gelöst, dessen Erfindung dadurch gekennzeichnet ist, daß der Verhältniszähler mit einer Torschaltung und diese mit einer internen Geräteüberwachungseinheit verbunden ist, an die durch die Torschaltung bei Unter- oder überschreiten einer eingestellten Tropfrate ein Signal abgebar ist, welches vorgebbare Funktionsüberwachungszyklen auslöst und daß die Geräteüberwachungseinheit mit einem Vorwahlzähler verbunden ist, der erst bei überschreiten einer vorgebbaren Anzahl von Funktionsüberwachungszyklen ein Signal an eine Warneinrichtung abgibt. Der Vorteil dieser Erfindung besteht darin, temporär auf das Schwerkraftinfusionsregelgerät einwirkende Artefakte (temporäre Behinderungen) von permanenten Behinderungen zu unterscheiden und herauszufiltern, ohne daß es zwingend notwendig zu einer sofortigen Alarmmeldung kommt. Durch die Geräteüberwachungseinheit wird die einmal registrierte Behinderung des Infusionsflusses in gegebenenfalls variablen Intervallen beliebig häufig auf Plausibilität hin überprüft und erst nach Ablauf einer ebenfalls vorgebbaren Anzahl von überprüfungszyklen eine Alarmmeldung ausgelöst. Hierdurch wird die Alarmhäufigkeit erheblich reduziert, das Pflegepersonal gleichermaßen entlastet und die Bereitschaft des Pflegepersonals erhöht, bei nunmehr mehr auftretenden Alarmmeldungen sofort zu handeln. Dies erhöht im wesentlichen Maße die Akzeptanz der Schwerkraftinfusionsregelgeräte bei Anwendern und Patienten.

Nach einer Weiterbildung der Erfindung ist die Geräteüberwachungseinheit mit einer Re-Start-Einrichtung verbunden, die bewirkt, daß der Infusionsflüssigkeitszufluß kurzzeitig unterbrochen und in wechselnder Weise wieder freigegeben wird, bis die überwachungseinrichtung den geforderten Soll-Stand der Infusionsflüssigkeit in der Tropfenkammer meldet. Vorzugsweise besteht zwischen dieser Re-Start-Einrichtung eine Verbindung zu einem Re-Start-Zähler, der wiederum mit dem bereits oben erwähnten Vorwahlzähler verbunden ist. Pendelt sich nach einer am Vorwahlzähler vorgebbaren Anzahl von Re-Start-Versuchen nicht der Normalfluß der Tropfeninfusion ein, wird zwangsläufig Alarm ausgelöst. Vorteilhaft ist beispielsweise eine Beschränkung auf sieben Re-Start-Phasen. Allerdings ist bei der Wahl der Re-Start-Phasen eine Medikamentenabhängigkeit des Patienten zu berücksichtigen. Die Anzahl der Re-Start-Phasen kann höher liegen, wenn ein langsam wirkendes Medikament zugeführt wird. Bei schnell wirkenden Medikamenten muß hingegen die Re-Start-Phase kurz sein, d.h. nur eine geringe Anzahl von Re-Start-Phasen ist tolerabel, um zu verhindern, daß beim Patienten eine Unterversorgung eintritt.

Nach einer weiteren Ausgestaltung der Erfindung ist der Vorwahlzähler wahlweise über einen Umschalter mit einer externen Warneinrichtung verbunden. Mit diesem Umschalter ist es beispielsweise möglich, zusätzlich zu der geräteinternen Warneinrichtung eine externe Warneinrichtung zuzuschalten, so daß auch außerhalb des Patientenzimmers, z.B. im Schwesternzimmer oder auf einer überwachungsstation,die Funktionsweise des Infusionsgerätes überwacht werden kann. Allerdings empfiehlt es sich häufig auch, nur über die externe Warneinrichtung einen Alarm auszulösen, um den Patienten nicht zu beunruhigen bzw. die übrigen, im Patientenzimmer befindlichen Personen nicht zu stören.

Vorzugsweise weist der zuvor genannte Umschalter mindestens zwei der nachfolgenden Schaltstellungsstufen auf:

a) Schaltverbindung vom Vorwahlzähler zur geräteinternen Warneinrichtung,

b) Schaltverbindung vom Vorwahlzähler zur externen Warneinrichtung und

c) Schalterverbindung vom Vorwahlzähler zu einem Zeitvorwahlschalter, der sowohl mit interner als auch externer Warneinrichtung, vorzugsweise wahlweise, verbindbar ist.

Mittels eines solchen Umschalters sind beispielsweise folgende Arbeitsvarianten bzw. Ansteuerungen der Warneinrichtung möglich:

Berücksichtigt man, daß bei Anlegen der Infusion der behandelnde Arzt oder das Pflegepersonal ohnehin im Zimmer anwesend ist und auch noch eine bewisse Zeit verbleibt, ist eine Alarmmeldung aus einer geräteinternen Warneinrichtung durchaus sinnvoll. Gleiches gilt für die Zeit und für solche Patienten, die eine ständige Anwesenheit von Pflegepersonal benötigen.

Verläßt die überwachungsperson das Patientenzimmer, besteht die Möglichkeit, auf die externe Warneinrichtung umzuschalten, so daß beispielsweise ein akustisches Warnsignal von dieser externen Warneinrichtung im Bedarfsfall abgegeben wird, womit nicht ausgeschlossen werden soll, daß optische Warnanzeigen an der geräteinternen Warneinrichtung nach wie vor angezeigt werden. Wählt man die Zeit-Vorwahleinstellung, so wird automatisch nach einer vorwählbaren Zeit die externe Warneinrichtung aktiviert und gleichzeitig die geräteinterne Warneinrichtung deaktiviert. Dieses Zeitglied beugt etwa vergessenen Umschaltungen auf die externe Warneinrichtung vor.

Eine weitere Schaltungsvariante wird ermöglicht, wenn die externe Warneinrichtung mit einem Ein-Aus-Schalter versehen wird, der das Abschalten der externen Warneinrichtung und gleichzeitige Einschalten der geräteinternen Warneinrichtung auslöst.

Nach einer weiteren Ausgestaltung der Erfindung ist die externe Warneinrichtung mit einem Zeitvorwahlschalter ausgerüstet, der nach Ablauf einer dort vorgebbaren Zeit die geräteinterne Warneinrichtung aktiviert. Dieser Zeitvorwahlschalter bzw. Regler kann auch mit der Ein-Aus-Schaltung der externen Warneinrichtung derart gekoppelt sein, daß nach einer gewissen Zeit sowohl die externe als auch die geräteinterne Warneinrichtung gleichermaßen bei Ansteuerung durch den Vorwahlzähler Alarm geben.

Wie bereits oben erwähnt, kann die geräteinterne oder die geräteexterne Warneinrichtung mit optischen und/oder akustischen Warnanzeigen versehen sein, die je nach Bedarf einzeln oder zusammen abschaltbar sind, z.B. dergestalt, daß zu Nachtzeiten nur die akustische externe Warnrichtung, an der geräteinternen Warneinrichtung jedoch nur eine optische Warneinrichtung,in Bereitschaft gesetzt werden.

Ein Ausführungsbeispiel der Erfindung ist in den Zeichnungen dargestellt. Es zeigen

Fig. 1 bis 5 ein Schwerkraftinfusionsregelgerät und

Fig. 6 in Blockdiagrammdarstellung eine schematische Darstellung der erfindungsgemäßen Anordnung (schematische Schaltskizze ).

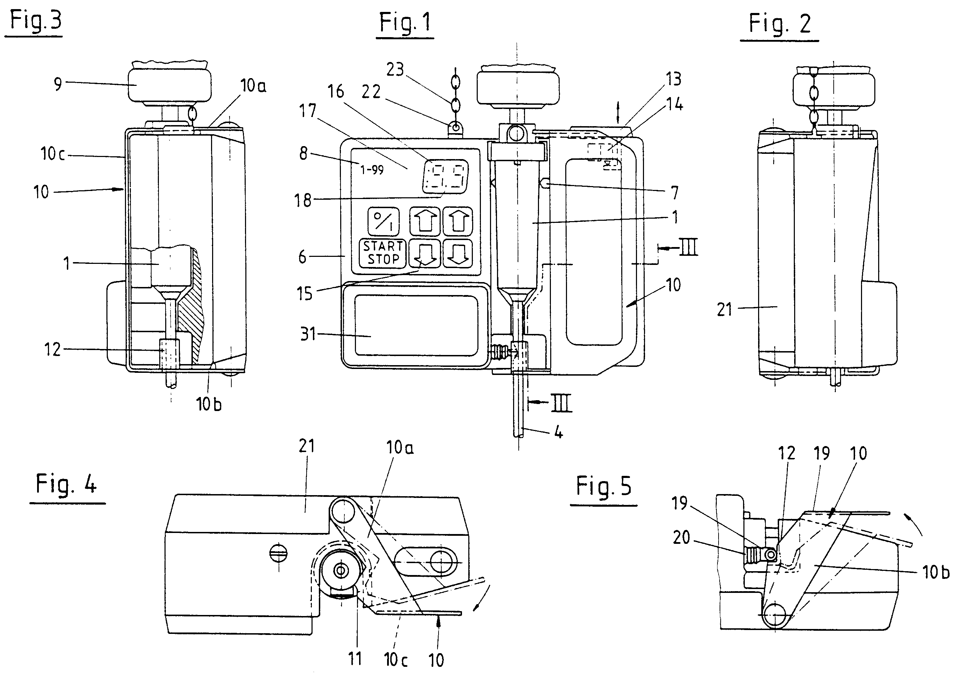

Das in den Fig. 1 bis 5 dargestellte Schwerkraftinfusionsregelgerät ist wie folgt ausgebildet:

Die bekannte zweiteilige, aus Tropfensensor und Regelgerät bestehende Bauweise ist durch einen monolithischen Aufbau ersetzt und in einem einzigen Gerätegehäuse sind eine fotoelektrische Tropfenerfassungseinrichtung 7, elektronische Einrichtungen mit den Bedienungselementen 8 und eine Regelmechanik eingeschlossen. Durch geeignete konstruktive Maßnahmen wird das Gewicht auf etwa 300 g begrenzt, damit das Gerätegehäuse direkt an die Tropfenkammer 1 angeklemmt werden kann, deren Einstichdorn so fest in dem Gummistopfen des Infusionsbehälters 9 eingeklemmt ist, daß er ohne weiteres 500 bis 600 g Zugbelastung aushalten kann.

Das Gerätegehäuse 6 weist in seinem Vorderteil eine den Konturen der Tropfenkammer 1 entsprechend ausgebildete Ausnehmung zur Aufnahme der Tropfenkammer 1 auf, wobei die Tropfenkammer 1 frontal einsetzbar ist.

An dem Gerätegehäuse 6 ist ein U-förmiger, unter der Einwirkung einer Feder stehender Hebel 10 angeordnet, dessen beide Schenkel 10a,10b obenseitig und untenseitig an dem Gerätegehäuse 6 verschwenkbar gelagert sind und dessen die beiden Schenkel 10a,10b miteinander verbindender Steg 10c an der Vorderseite des Gerätes als Handhabe liegend ist, wobei der obere Schenkel 10a des Hebels 10 in einer Ausnehmung 11 mit einer der Tropfenkammer entsprechenden Formgebung zur Klemmung und Halterung der Topfenkammer 1 versehen ist, während der untere Schenkel 10b des Hebels 10 ein mittels des Hebels verschwenkbares, zur Anlage an dem eingelegten Infusionsschlauch 4 bringbares Widerlager 12 zum ungehinderten Einlegen des mit der Tropfenkammer 1 verbundenen Infusionsschlauches 4 bei gedrückter Hebelstellung und geöffnetem Widerlager und für die Quetschung des Infusionsschlauches 4 durch ein Regelschwert 19 bei Normal-Hebelstellung aufweist, wobei der Hebel 10 gegen ein unbeabsichtigtes Betätigen mit einer knopfartigen, an dem oberen Hebelschenkel 10a anliegenden Sperre 13 in Wirkverbindung steht, die vor dem Verschwenken des Hebels 10 betätigbar ist. Damit der Hebel 10 nicht unbeabsichtigt betätigt werden kann, mit der wahrscheinlichen Folge eines Freiflusses muß erst die knopfartige Sperre 13 gedrückt werden, bevor der Hebel 10 geschwenkt werden kann.

Diese Sperre 13 dient gleichzeitig durch Betätigung eines Mikroschalters 14 als Meldeelement für die richtige Lage des Hebels 10 und konsequenterweise auch für die korrekte Montage der Tropfenkammer 1 und des Infusionsschlauches 4, damit die elektronische Einrichtung im Fall von Bedienungsfehlern Alarm melden kann.

Das an dem Gerätegehäuse 6 vorgesehene Tastenfeld 15 ist aus Platzgründen vereinfacht ausgeführt und seine Arbeitsweise ist wie die eines durch Drucktasten zu betätigenden Dekodierschalters. Eine LCD-Anzeige 16 der Tropfrate, die ständig oder mit verschiedenen Frequenzen leuchtet, enthält auch die Batterieladezustandsanzeige 17 und meldet durch Blinken des Dezimalpunktes 18 das Fallen jedes Tropfens.

Die fotoelektrische Tropfenerfassung 7, bestehend aus einer Leuchtdiode (LED) und einem Fototransistor, ist in das Gerätegehäuse 6 integriert, sowie das durch einen Minischrittmotor angetriebene Getriebe so ausgelegt, daß die jeweils erforderlichen Sicherungsanforderungen erfüllt sind, wobei durch eine Nocke das Regelschwert 19 betätigt wird, um den Okklusionsgrad variieren zu können. Das Gerät ist durch geeignete Maßnahmen, wie ein Balg 20 und andere geeignete Dichtelemente gegen Schwallwasser geschützt und somit leicht zu reinigen, sowie gegen eventuelles Auslaufen der Infusionslösung.

Mit 21 ist ein Batteriekasten bezeichnet, der NiCD-Elemente enthält, der zwecks leichter Auswechselbarkeit mittels Permanent-Magneten mit relativen Gegenpolen in den vorgesehenen Gehäuseaussparungen gehalten wird, und der so bemessen ist, daß mindestens 24 Stunden Batteriebetrieb gewährleistet ist. Ein zweiter Batteriekasten wird während dieser Zeit in einem separaten Ladegerät in etwa 14 Stunden vollgeladen, so daß ein ununterbrochener Betrieb mit ausreichender Sicherheitsmarge gewährleistet ist. Durch geeignete Auswahl der elektronischen Bauteile und der Anzeigeelemente ist die Schaltung so ausgelegt, daß der Stromverbrauch minimiert wird. In einer Öse 22 ist eine Kette 23 angebracht, die am Flaschenhalter befestigt werden kann, um ein versehentliches Herunterfallen des Gerätes zu vermeiden.

Solche Einrichtungen sind beispielsweise aus der EP 0 154 163 bekannt.

Teil des Gerätegehäuses 6 des Schwerkraftinfusionsregelgerätes ist ein Verhältniszähler 23, der die Tropfrate pro Zeiteinheit mißt und der eine Torschaltung und einen Zeitbasisgenerator enthält. Dieser Verhältniszähler 23 ist mit einer Torschaltung 24 verbunden bzw. gibt an diese Impulse ab, wobei die Torschaltung bei Unter- oder überschreiten einer eingestellten Tropfrate bzw. eines Bereiches ein Signal an eine Geräteüberwachungseinheit 25 abgibt, die Gerätefunktionsüberwachungszyklen auslöst und gleichzeitig eine Re-Start-Einrichtung 26 aktiviert. Sowohl die Anzahl der Re-Start-Aktivierungen als auch die der Funktionsüberwachungszyklen werden durch einen Re-Start-Zähler 27 und einen Vorwahlzähler 28 erfaßt, wobei der Vorwahlzähler 28 erst nach einer vorgebbaren Anzahl von überwachungszyklen bzw. Re-Start-Versuchen, z.B. 7, über die Leitung 29 einen Impuls abgibt. Dieser Impuls wird je nach Stellung des Umschalters 30 über eine der Leitungen 29a,29b oder 20c letztlich an die geräteinterne Warneinrichtung 31 oder die geräteexterne Warneinrichtung 32 weitergegeben, wo gleichzeitig oder nur von einer Einrichtung ein Alarmsignal abgegeben wird. Wählt man die obere Schaltstellung, erhält man ausschließlich ein Alarmsignal von der geräteinternen Warneinrichtung 31, und zwar dann und nur dann, wenn am Vorwahlzähler 28 eine bestimmte Anzahl, wie von Geräten 25 oder 27 gemeldet, erreicht wird. Bei der mittleren Schaltstellung des Umschalters 30 wird (zunächst) nur die externe Warneinrichtung 32 in Bereitschaftsstellung versetzt. Bei der dritten, unteren Schaltstellung wird der Impuls über die Leitung 29c auf einen Zeitvorwahlschalter 33 gegeben, der vor Ablauf der eingestellten Umschaltzeit das die Warnung auslösende Signal über die Leitung 34 auf die geräteinterne Warneinrichtung 31 gibt und nach Ablauf dieser Zeit über die Leitung 35 auf die externe Warneinrichtung 32 . Diese Schaltungsvariante schließt gegebenenfalls auch die Möglichkeit ein, daß während der gesamten überwachungszeit an der geräteinternen Warneinrichtung 31 eine optische Warnanzeige erfolgt, während die akustische Warnanzeige an der Warneinrichtung 32 erst nach Ablauf der Umschaltzeit ausgelöst wird. Die externe Warneinrichtung 32 besitzt ferner noch einen Ein-Aus-Schalter 36 sowie einen Zeitvorwahlschalter 37, die folgendermaßen arbeiten: Betätigt man den Ein-Aus-Schalter 36 an der externen Warneinrichtung 32, so wird das Warnsignal auf die geräteinterne Warneinrichtung umgeschaltet. Der Zeitvorwahlschalter 37 hingegen aktiviert nach vorgebbarer Zeiteinstellung die geräteinterne Warneinrichtung 31, so daß gegebenenfalls die Warneinrichtungen 31 und 32 parallel Warnsignale abgeben. Gegebenenfalls kann jedoch der Zeitvorwahlschalter 37 auch den Ein-Aus-Schalter 36 aktivieren.

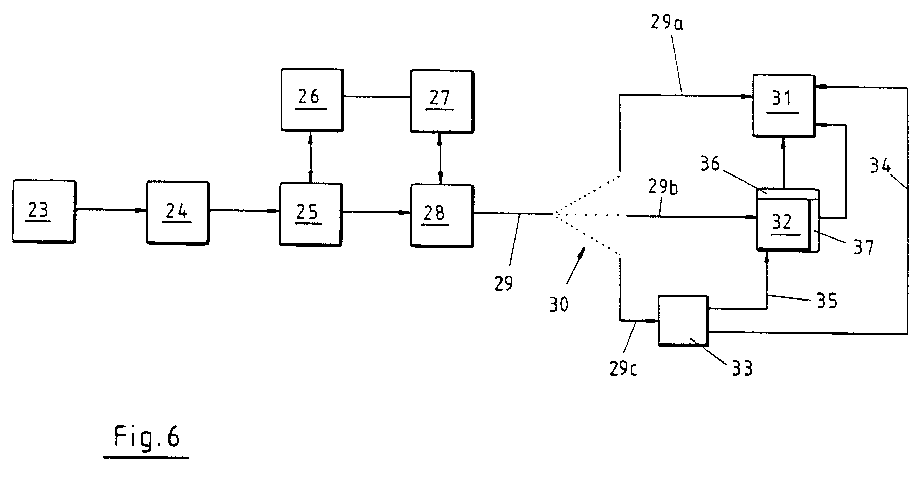

Die in Fig. 6 dargestellte Schaltung basiert auf ECD-Schaltungen, wie sie beispielsweise durch Flip-Flops realisiert werden können. Gleichermaßen kann jedoch eine solche Schaltung auch in Monolith-Bauweise über eine Transistor-Logik, gegebenenfalls in einer Modulform,realisiert werden. Wesentlicher Gedanke ist jeweils, daß ein oder mehrere Warnsignale nur nach Ausfilterung kurzzeitiger Störungen ausgelöst werden.

**Patentansprüche**

1. Schwerkraftinfusionsregelgerät, das aus einem Gerätegehäuse (6) mit einer Aufnahmekammer für eine Tropfenkammer (1) besteht, eine im Bereich der Tropfenkammer (1) angeordnete fotoelektrische Tropfenerfassungseinrichtung (7) mit einem Verhältniszähler (23) zur Registrierung der Tropfrate pro Zeiteinheit und eine elektronische überwachungseinrichtung mit Bedienungselementen sowie eine Regelmechanik aufweist, dadurch gekennzeichnet, daß der Verhältniszähler (23) mit einer Torschaltung (24) und diese mit einer internen Geräteüberwachungseinheit (25) verbunden sind, an die durch die Torschaltung (24) bei Unter- oder überschreiten einer eingestellten Tropfrate ein Signal abgebbar ist, welches vorgebbare Funktionsüberwachungszyklen auslöst und daß die Geräteüberwachungseinheit (25) mit einem Vorwahlzähler (28) verbunden ist, der erst bei überschreiten einer vorgebbaren Anzahl von Funktionsüberwachungszyklen ein Signal an eine Warneinrichtung (31,32) abgibt.

2. Schwerkraftinfusionsregelgerät nach Anspruch 1, dadurch gekennzeichnet, daß die Geräteüberwachungseinheit (25) mit einer Re-Start-Einrichtung (26) verbunden ist.

3. Schwerkraftinfusionsregelgerät nach Anspruch 2, dadurch gekensnzeichnet, daß die Re-Start-Einrichtung (26) einen Re-Start-Zähler (27) aufweist, der mit dem Vorwahzähler (28) verbunden ist.

4. Schwerkraftinfusionsregelgerät nach einem der Ansprüche 1 bis 3, dadurch gekennzeichnet, daß der Vorwahlzähler (28) wahlweise über einen Umschalter (30) mit einer externen Warneinrichtung (32) verbunden ist.

5. Schwerkraftinfusionsregelgerät nach Anspruch 4, dadurch gekennzeichnet, daß der Umschalter (30) mindestens zwei der nachfolgenden Schaltstellungsstufen aufweist:
   a) Schaltverbindung vom Vorwahlzähler (28) zur geräteinternen Warneinrichtung (31),
   b) Schaltverbindung vom Vorwahlzähler (28) zur externen Warneinrichtung (32),
   c) Schaltverbindung vom Vorwahlzähler (28) zu einem Zeitvorwahlschalter (33), der sowohl mit der geräteinternen als auch der externen Warneinrichtung (31,32) verbunden ist.

6. Schwerkraftinfusionsregelgerät nach Anspruch 5, daqdurch gekennzeichnet, daß die externe Warneinrichtung (32) über einen Zeitvorwahlschalter (37 mit der geräteinternen Warnrichtung (31) verbunden ist, wobei der Zeitvorwahlschalter (37) vorzugsweise mit einem Ein-Aus-Schalter (36) der externen Warneinrichtung (32) ausschaltbar ist.

7. Schwerkraftinfusionsregelgerät nach einem der Ansprüche 2 bis 6, dadurch gekennzeichnet, daß die externe Warneinrichtung (32) einen Ein-Aus-Schalter (36) besitzt, der mit der internen Warneinrichtung in umkehrender Schaltlogik verbunden ist.

8. Schwerkraftinfusionsregelgerät nach einem der Ansprüche 1 bis 7, dadurch gekennzeichnet, daß die externe und/oder geräteinterne Warneinrichtung (31,32) eine optische und/oder akustische Warnanzeige aufweisen.

Fig. 1

Fig. 2

Fig. 3

Fig. 4

Fig. 5

Fig.6